(19)

**Europäisches Patentamt**

**European Patent Office**

**Office européen des brevets**

(11) **EP 3 344 219 B1**

(12) **EUROPEAN PATENT SPECIFICATION**

(45) Date of publication and mention
of the grant of the patent:
**02.12.2020 Bulletin 2020/49**

(21) Application number: **17737129.1**

(22) Date of filing: **23.06.2017**

(51) Int Cl.:
*A61K 8/27* (2006.01)          *A61K 8/44* (2006.01)
*A61Q 11/00* (2006.01)        *A61K 8/73* (2006.01)
*A61K 8/24* (2006.01)

(86) International application number:
**PCT/US2017/039074**

(87) International publication number:
**WO 2017/223493 (28.12.2017 Gazette 2017/52)**

(54) **ORAL CARE COMPOSITIONS AND METHODS OF USE**

MUNDPFLEGEZUSAMMENSETZUNGEN UND VERFAHREN ZUR VERWENDUNG

COMPOSITIONS DE SOINS BUCCAUX ET PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.06.2016 PCT/CN2016/086994**

(43) Date of publication of application:
**11.07.2018 Bulletin 2018/28**

(73) Proprietor: **Colgate-Palmolive Company
New York, NY 10022 (US)**

(72) Inventors:
• **POTANIN, Andrei
Hillsborough, NJ 08844 (US)**
• **POTH, Tilo
69469 Weinheim (DE)**
• **AHUJA, Amit
Highland Park, NJ 08904 (US)**
• **BLANVALET, Claude
B-4031 Angleur (BE)**
• **WON, Betty
Princeton Junction, NJ 08550 (US)**
• **MANUS, Lisa
Lawrenceville, NJ 08648 (US)**
• **STRANICK, Michael, A.
Bridgewater, NJ 08807 (US)**
• **HUANG, Xiao Yi
Guangzhou City
Guangdong 510730 (CN)**
• **PRENCIPE, Michael
West Windsor, NJ 08550 (US)**

• **RUSSO, Amy
Belle Mead, NJ 08502 (US)**
• **STETTLER, Hansruedi
CH-4054 Basel (CH)**
• **YAN, Peng
Guangzhou City
Guangdong 510730 (CN)**
• **TAN, Chengkang
Guangzhou City
Guangdong 510730 (CN)**
• **PATEL, Vyoma
Hillsborough, NJ 08844 (US)**
• **MORGAN, Andre, Michelle
Robbinsville, NJ 08690 (US)**

(74) Representative: **Sonnenhauser, Thomas Martin
Wuesthoff & Wuesthoff
Patentanwälte PartG mbB
Schweigerstraße 2
81541 München (DE)**

(56) References cited:
**WO-A1-2015/094152          WO-A1-2015/094849
JP-A- H07 304 641            JP-A- 2011 105 682
US-A1- 2007 224 134          US-A1- 2010 135 921
US-A1- 2015 320 654**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 344 219 B1

- **H Hx ET AL: "PRODUCT DATA Ashland Specialty Ingredients Types and Specifications C, mPa.s (Method N5-5) TYPES Viscosity measured at a concentration of Brookfield LVF setting Non-R X W and D R B 1% 2% 5% Spindle no", Ashland. Rev, 1 January 2011 (2011-01-01), pages 5-2015, XP55395554, Retrieved from the Internet: URL:http://www.brenntag.com/media/documents/bsi/product_data_sheets/material_science /ashland_cellulose_rheology_modifiers/natr osol_250_pds.pdf**

Remarks:

The file contains technical information submitted after the application was filed and not included in this specification

**Description**

BACKGROUND

Arginine-based oral care compositions generally include some combination of polymers, abrasive(s); and in some instances, additional active ingredients. In those instances where additional active ingredients are included and comprise cationic metal ions, e.g. zinc, maintaining the physical stability of the composition is a challenge because of the interaction between these cationic metal ions and certain polymeric components and abrasive systems.

The use of certain abrasives or specific concentrations of particular polymers are two ways in which the stability issues have been addressed. However, these methods have generally focused individually on stand-up (i.e., appearance on the brush) and squeezability from packaging (toothpaste tubes). As such, there remains a need to reconcile these physical stability and rheological concerns. Certain embodiments of the present invention are designed to address this need.

US 2015/320654 (A1) discloses an oral care composition comprising: a. from 0.01% to 5% by weight of a stannous ion source; b. from 0.01% to 15% by weight of a thickener comprising at least 2 agents selected from the group consisting of: i) a linear sulfated polysaccharide; ii) a natural gum; and iii) a non-ionic cellulose derivative; and c. at least 30% by weight of a total water content; wherein the composition has a pH between 4 to 10.

BRIEF SUMMARY

Some embodiments of the present invention provide oral care compositions comprising a basic amino acid in free or salt wherein the amino acid is selected from arginine, lysine, and a combination thereof; a combination of zinc ion sources; and a thickening system as defined in the claims.

Other embodiments provide compositions further comprising a silica abrasive which exhibits an approximately neutral pH when measured in an aqueous medium. Still further embodiments provide oral care compositions comprising a basic amino acid in free or salt wherein the amino acid is selected from arginine, lysine, and a combination thereof; a combination of zinc ion sources; a thickening system as defined in the claims; and a silica abrasive which exhibits an approximately neutral pH when measured in an aqueous medium.

In some embodiments, the oral care compositions of the present invention demonstrate the ability to avoid viscosity loss and maintain static yield stress over an extended period of time, e.g. after one year.

In one aspect the invention, thus, is an oral care composition comprising:

> a. a basic amino acid in free or salt form wherein the basic amino acid is selected from arginine, lysine, and a combination thereof;
> b. a combination of zinc ion sources; and
> c. a thickening system comprising:
>
> > i. from 0.1 wt.% to 0.4 wt.% of a nonionic cellulose ether which is hydroxyethylcellulose; and
> > ii. from 0.25 wt.% to 0.9 wt.% of a polysaccharide gum which is xanthan gum.

In another embodiment, the invention encompasses said oral care compositions of the invention for use in a method as defined in the claims.

DETAILED DESCRIPTION

As used herein, the terms "oral composition" and "oral care composition" refer to the total composition that is delivered to the oral surfaces. The composition is further defined as a product which, during the normal course of usage, is not intended for systemic administration of particular therapeutic agents or intentionally swallowed; but rather, is retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition is provided as a dual phase composition, wherein individual compositions are combined when dispensed from a separated compartment dispenser.

Basic Amino Acids

In certain embodiments, the basic amino acid is arginine, for example, L-arginine, or a salt thereof.

The compositions of the invention are intended for topical use in the mouth and so salts for use in the present invention should be safe for such use, in the amounts and concentrations provided. Suitable salts include salts known in the art to be pharmaceutically acceptable salts which are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources used with the present invention include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

Surfactants

The invention may in some embodiments contain anionic surfactants, for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium coco-glyceride sulfate; higher alkyl sulfates, such as sodium lauryl sulfate; higher alkyl-ether sulfates, e.g., of formula $CH_3(CH_2)_mCH_2(OCH_2CH_2)_nOSO_3X$, wherein m is 6-16, e.g., 10, n is 1-6, e.g., 2, 3 or 4, and X is Na or, for example sodium laureth-2 sulfate $(CH_3(CH_2)_{10}CH_2(OCH_2CH_2)_2OSO_3Na)$; higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., $C_{6-30}$ alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

Cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from polaxamers (e.g., polaxamer 407), polysorbates (e.g., polysorbate 20), polyoxyl hydrogenated castor oils (e.g., polyoxyl 40 hydrogenated castor oil), and mixtures thereof.

Illustrative amphoteric surfactants that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

Illustrative zwitterionic surfactants that can be used in the compositions of the invention include derivatives of aliphatic quaternary ammonium, phosphonium and sulfonium compounds in which the aliphatic radical can be a straight or branched chain and wherein one of the aliphatic substituents contains about 8-18 carbon atoms and one contains an anionic water-solubilizing group (such as carboxy, sulfonate, sulfate, phosphate or phosphonate). The surfactant or mixtures of compatible surfactants can be present in the compositions of the present invention in 0.1% to 5%, in another embodiment 0.3% to 3% and in another embodiment 0.5% to 2% by weight of the total composition.

Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide least 0.1 wt. % pyrophosphate ions, e.g., 0.1 to 3 wt 5, e.g., 0.1 to 2 wt %, e.g., 0.1 to 1 wt%, e.g., 0.2 to 0.5 wt%. The pyrophosphates also contribute to preservation of the compositions by lowering the effect of water activity.

Polymers

The oral care compositions of the invention also optionally include one or more further polymers, such as polyethylene glycols, or polyvinyl methyl ether maleic acid copolymers. Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorosorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

In preparing oral care compositions, it is sometimes necessary to add some thickening material to provide a desirable consistency or to stabilize or enhance the performance of the formulation. According to the invention, as thickening system as defined in the claims is used.

## Abrasives

Generally, the inclusion of abrasives in dentifrice formulations is necessary for effective cleaning of teeth by brushing. It has been determined that by including an abrasive silica having an acid pH in the composition, compositions of enhanced viscosity stability are obtained. Prophy silica available from Grace, offered as Sylodent™, can be used with various embodiments of the present invention.

The acidic silica abrasive is included in the dentifrice components at a concentration of about 2 to about 35% by weight; about 3 to about 20 % by weight, about 3 to about 15% by weight, about 10 to about 15 % by weight. For example, the acidic silica abrasive may be present in an amount selected from 2 wt.%, 3 wt.%, 4% wt.%, 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, 20 wt.%.

A commercially available acidic silica abrasive is Sylodent 783 available from W. R. Grace & Company, Baltimore, Md. Sylodent 783 has a pH of 3.4-4.2 when measured as a 5% by weight slurry in water. For use in the present invention, the silica material has an average particle size of less than 10 microns, e.g., 3-7 microns, e.g. about 5.5 microns. For example a small particle silica may have an average particle size (D50) of 2.5 - 4.5 microns.

The composition may also include any silica suitable for oral care compositions, such as precipitated silicas or silica gels. For example synthetic amorphous silica. Silica may also be available as a thickening agent, e.g., particle silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

The invention may also comprise a commercially available cleaning silica in certain embodiments of the invention. Zeodent 114 offered by J.M. Huber Finland Oy Telakkatie 5 FIN-49460 Hamina, is one such commercially available silica.

## Water

Water is present in the oral compositions of the invention. Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

## Humectants

Within certain embodiments of the oral compositions, it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerin, sorbitol, xylitol, propylene glycol as well as other polyols and mixtures of these humectants. Mixtures of glycerin and sorbitol may be used in certain embodiments as the humectant component of the compositions herein.

The oral care compositions are for use in methods as defined in the claims, wherein the method involves applying to the oral cavity a safe and effective amount of the compositions described herein.

The compositions according to the invention can be incorporated into oral compositions for the care of the mouth and teeth such as toothpastes, transparent pastes, gels, mouth rinses, sprays and chewing gum.

Some embodiments provide compositions comprising a nonionic cellulose ether having a molecular weight of from about 650,000 to about 750,000. Other embodiments provide compositions comprising a nonionic cellulose ether having a molecular weight of about 700,000. While other embodiments provide compositions comprising a nonionic cellulose ether having a molecular weight of about 720,000.

Embodiments provide oral care compositions comprising about 0.1 wt.%, about 0.15 wt.%, about 0.2 wt.%, about 0.25 wt.%, about 0.3 wt.%, about 0.35 wt.%, about 0.4 wt.%, about 0.45 wt.% or about 0.5 wt.% of hydroxyethylcellulose. Certain embodiments provide oral care compositions comprising 0.1 wt.%, 0.15 wt.%, 0.2 wt.%, 0.25 wt.%, 0.3 wt.%, 0.35 wt.%, or 0.4 wt.% of hydroxyethylcellulose.

In some embodiments, the hydroxyethylcellulose has a viscosity, measured at 2% in water at 25 °C, of about 4500 to about 7500 cps. In some embodiments, the hydroxyethylcellulose has a viscosity, measured at 2% in water at 25 °C, of about 4500 to about 6500 cps. In some embodiments, the hydroxyethylcellulose has a viscosity, measured at 2% in water at 25 °C, of about 6000 to about 7500 cps.

In some embodiments, the hydroxyethylcellulose having a viscosity, measured at 2% in water at 25 °C, of about 4500 to about 6500, is present in an amount of from about 0.1 wt.% to about 1 wt.%, of the oral care composition. In some embodiments, the hydroxyethylcellulose having a viscosity, measured at 2% in water at 25 °C, of from about 4500 to about 6500 cps, is present in an amount of from about 0.1 wt.% to about 0.5 wt.%, of the total composition. In some embodiments, the hydroxyethylcellulose having a viscosity, measured at 2% in water at 25 °C, of about 4500 to about 6500 cps, is present in an amount of about 0.1 wt.%, about 0.15 wt.%, about 0.2 wt.%, about 0.25 wt.%, about 0.3 wt.%, about 0.35 wt.%, about 0.4 wt.%, about 0.45 wt.% or about 0.5 wt.% of the oral care composition.

Some embodiments provide oral care compositions comprising hydroxyethylcellulose having a molecular weight of about 700,000, e.g. 720,000. Other embodiments provide oral care compositions comprising 0.1 wt.%, 0.15 wt.%, 0.2 wt.%, 0.25 wt.%, 0.3 wt.%, 0.35 wt.%, or 0.4 wt.% of a hydroxyethylcellulose having a molecular weight of about 700,000, e.g. 720,000.

Some embodiments provide oral care compositions comprising hydroxyethylcellulose having a molecular weight of about 350,000. Other embodiments provide oral care compositions comprising 0.1 wt.%, 0.15 wt.%, 0.2 wt.%, 0.25 wt.%, 0.3 wt.%, 0.35 wt.%, or 0.4 wt.% of a hydroxyethylcellulose having a molecular weight of about 350,000.

According to the invention, the polysaccharide gum is xanthan gum. Embodiments provide oral care compositions comprising from 0.6 wt.% to 0.9 wt.% of xanthan gum. In further embodiments, the oral care composition comprises from 0.7 wt.% to 0.8 wt.% of xanthan gum. Still further embodiments provide oral care compositions comprising about 0.3 wt.%, 0.4 wt.% 0.5 wt.%, 0.6 wt.%, 0.7 wt.%, 0.8 wt.%, or 0.9 wt.% of xanthan gum.

In some embodiments, the thickening system further comprises from about 5 wt.% to about 10 wt.% silica. In some embodiments, the thickening system comprises from about 0.5 wt.% to about 15 wt.% of the oral care composition.

In certain embodiments, the hydroxyethylcellulose and the polysaccharide gum are present in a weight ratio of from about 1:1 to about 1:10. In other embodiments, the hydroxyethylcellulose and the polysaccharide gum are present in a weight ratio of from about 1:2 to about 1:9. Yet other embodiments provide compositions wherein the hydroxyethylcellulose and the polysaccharide gum are present in a weight ratio of from about 1:3 to about 1:7.

In some embodiments, the oral care composition further comprises a fluoride ion source selected from sodium fluoride, sodium monofluorophosphate, and stannous fluoride.

In some embodiments, the oral care composition comprises about 1.0 wt.% zinc oxide; about 0.5 wt.% zinc citrate; about 1.5 wt.% L-arginine; from about 0.3 wt.% to about 1 wt.% of xanthan gum; and from about 0.1 wt.% to about 1 wt.% of hydroxyethylcellulose. Some embodiments comprise from about 0.6 wt.% to about 0.9 wt.% of xanthan gum. Some embodiments comprise from about 0.7 wt.% to about 0.8 wt.% of xanthan gum.

In further embodiments, the oral care composition loses no more than about 45% of its initial viscosity after one year. In some embodiments, the oral care composition loses no more than about 40% of its initial viscosity after one year. In other embodiments, the oral care composition loses no more than about 35% of its initial viscosity after one year. In yet other embodiments, the oral care composition loses no more than about 30% of its initial viscosity after one year. In some embodiments, the oral care composition loses no more than about 25%, 24%, 23%, 22%, 21% or 20% of its initial viscosity after one year.

In some embodiments, the oral care composition has a G'/G" ratio of greater than 0.5. In some embodiments, the oral care composition has a G'/G" ratio of greater than 0.75. In some embodiments, the oral care composition has a G'/G" ratio of greater than 1. In some embodiments, the oral care composition has a G'/G" ratio of greater than 1.5. In some embodiments, the oral care composition has a G'/G" ratio of less than 2. In some embodiments, the oral care composition has a G'/G" ratio of less than 1.5. In some embodiments, the oral care composition has a G'/G" ratio of less than 1. Methods of quantifying the elastic modulus (G'), the loss modulus (G") and G'/G" ratios are described, for example, in WO 2013/089734 A1.

In some embodiments, the compositions of the present invention provide a consistency, K, less than 30 $Pa*s^n$. In some embodiments, the compositions of the present invention provide a flow index, n, of greater than 0.3. In some embodiments, the compositions of the present invention provide a consistency, K, less than 30 $Pa*s^n$ and a flow index, n, of greater than 0.3. In some embodiments, the compositions of the present invention provide a consistency, K, less

than 30 Pa*s$^n$; a flow index, n, of greater than 0.3; and a G'/G" ratio of less than 2. In some embodiments, the compositions of the present invention provide a flow index, n, of greater than 0.3; and a G'/G" ratio of less than 2. In some embodiments, the compositions of the present invention provide a consistency, K, less than 30 Pa*s$^n$; and a G'/G" ratio of less than 2.

In some embodiments, the oral care compositions of the present invention provide a yield stress greater than 20 Pa. In some embodiments, the oral care compositions of the present invention provide a yield stress greater than 25 Pa. In other embodiments, the oral care compositions of the present invention provide a yield stress greater than 30 Pa. Yet further embodiments, provide oral care compositions that demonstrate a yield stress greater than 35 Pa. In some embodiments, the oral care compositions of the present invention provide a yield stress greater than 40 Pa.

Some embodiments provide compositions having a drainage time of less than about 10 minutes when draining 3 kg of an intermediate product (gel pre-mix) from a tank of 21 cm in diameter through a 1 mm wide bottom opening at negative pressure of - 0.95 bar. Other embodiments provide compositions having a drainage time of less than about 9 minutes. Further embodiments provide compositions having a drainage time of less than about 8 minutes. Yet other embodiments provide compositions having a drainage time of less than about 7 minutes, 6 minutes, 5 minutes, 4 minutes, 3 minutes, 2 minutes or 1 minute.

Some embodiments provide compositions demonstrating less than 300 grams of left-overs. Other embodiments provide compositions demonstrating less than 275 grams of left-overs. Further embodiments provide compositions demonstrating less than 250 grams of left-overs. Yet other embodiments provide compositions demonstrating less than 225 grams of left-overs. Still further embodiments provide compositions demonstrating less than 200 grams of left-overs. While other embodiments provide compositions demonstrating less than 175 grams, 150 grams, 125 grams, 100 grams or 50 grams of left-overs. Certain embodiments provide compositions demonstrating a drainage time of less than 10 minutes and less than 225 grams of left-overs.

As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention.

EXAMPLES

Example 1

The examples herein detail how the viscosity over time for a composition which exhibits a problem of rapid reduction in viscosity (Run A), is compared to five compositions which show the stabilized viscosity provided by the invention (Compositions 1-5 in Table 1).

Viscosity is measured on a Brookfield HADV2 viscometer using a V74 vane spindle. This viscometer applies a user-controlled angular velocity to the spindle, typically measured in rotations per second (RPM), and reports torque on the shaft of the spindle. Viscosity is then calculated from RPM and torque as explained in the Brookfield Manual (Operating Instructions) using too conversion parameters SRC (shear rate constant) and SMC (spindle multiplier constant). The conversion parameters are defined as follows: SMC=290, SRC=0.2723. The test is performed at room temperature, and varies between 22 and 25 °C. During the test, RPM of the spindle is swept from 200 to 0.5 in 12 steps, 10 seconds per step. The viscosity reading reported is taken at RPM=1.

Example 2

Table 5 (below) describes the formulas of Compositions 9, 10 and 11 (not claimed) and a further comparative example (Comparative Example I).

Table 5

| | Composition 9 | Composition 10 | Composition 11 | | Comparative Example I |
|---|---|---|---|---|---|
| Ingredient | Wt% | | | | |
| GLYCERIN | 35.00000 | 26.00000 | 26.00000 | | 35.00000 |
| SORBITOL | -- | 13.00000 | 13.00000 | | -- |

(continued)

| | Composition 9 | Composition 10 | Composition 11 | | Comparative Example I |
|---|---|---|---|---|---|
| Ingredient | | | Wt% | | |
| WATER | 30.90474 | 27.82474 | 27.62474 | | 30.84874 |
| SILICA ABRASIVE 1 | 10.00000 | 10.00000 | 10.00000 | | 10.00000 |
| SILICA- THICKENER | 7.00000 | 6.00000 | 6.00000 | | 7.00000 |
| SILICA ABRASIVE 2 | 5.00000 | 5.00000 | 5.00000 | | 5.00000 |
| Na-LAURYL SULFATE GRANULES | 2.10526 | 2.10526 | 2.10526 | | 2.10526 |
| Flavor | 1.50000 | 1.50000 | 1.50000 | | 1.50000 |
| L-ARGININE | 1.50000 | 1.50000 | 1.50000 | | 1.50000 |
| COCAMIDOPROPYL BETAINE | 1.25000 | 1.25000 | 1.25000 | | 1.25000 |
| ZINC OXIDE | 1.00000 | 1.00000 | 1.00000 | | 1.00000 |
| TITANIUM DIOXIDE | 0.75000 | 0.75000 | 0.75000 | | -- |
| XANTHAN GUM | 0.60000 | 0.30000 | 0.30000 | | 0.40000 |
| SODIUM CMC | -- | -- | -- | | 1.10000 |
| HYDROXYETHYLCELLULOSE (HEC)* | 0.50000 | 0.80000 | 1.00000 | | -- |
| TETRA S ODIUM PYROPHOSPHATE | 0.50000 | 0.50000 | 0.50000 | | 0.50000 |
| ZINC CITRATE TRIHYDRATE | 0.50000 | 0.50000 | 0.50000 | | 0.50000 |
| POLOXAMER 407 | 0.50000 | 0.50000 | 0.50000 | | 0.50000 |
| BENZYL ALCOHOL | 0.40000 | 0.40000 | 0.40000 | | 0.40000 |
| 85% PHOSPHORIC ACID | 0.35000 | 0.35000 | 0.35000 | | 0.35000 |
| SODIUM FLUORIDE - USP, EP | 0.32000 | 0.32000 | 0.32000 | | 0.32000 |
| Sweeteners | 0.32000 | 0.40000 | 0.40000 | | 0.42000 |
| Additional Colorants | -- | -- | -- | | 0.30600 |
| *HEC having a viscosity, measured at 2% in water at 25 °C, of 6000 to 7500 cps | | | | | |

Example 3

Table 6 (below) describes the results of viscosity and static yield stress evaluations performed on an exemplary composition and a reference formula.

Viscosity and Yield Stress are measured on a Brookfield HADV2 viscometer using V74 vane spindle 1.176 cm in length and 0.589 cm in diameter. This viscometer applies a user-controlled angular velocity to the spindle, typically measured in rotations per second (RPM), and reports torque, T%, measured in the percentage of the maximum total torque on the shaft of the spindle. The torque, T, measured in SI units, N*m, is related to T% as reported by the above mentioned viscometer as $T=1.437*10^{-5}*T\%$.

The tests are performed at room temperature (22 to 25 °C). During the test 0.5 RPM of the spindle is first rotated for 400 sec and then RPM is swept from 0.5 to 200 and back to 0.5 in 12 logarithmical steps each way, 10 seconds per step. The viscosity reading is taken at RPM=1 on the decreasing RPM sweep. Viscosity is then calculated from RPM and T as explained in the Brookfield Manual (Operating Instructions) using two conversion parameters SRC (shear rate constant) and SMC (spindle multiplier constant). In this case, the conversion parameters are defined as follows: SMC=290, SRC=0.27.

Static Yield Stress (YS) is calculated as a fitting parameter by fitting experimental T(RPM) dependence on increasing RPM sweep with the theoretical one which was calculated assuming the so-called Casson constitutive equation and is implicitly given by the following equation:

$$RPM = \frac{15}{\pi} \int_{YS}^{SW} \frac{\left(S^n - YS^n\right)^{1/n}}{HSV * S} dS$$

where HSV (high-shear viscosity limit) is another fitting parameter, n=0.3, and SW is the stress on the imaginary wall encompassing the vane which is estimated as follows:

$$SW = \frac{2T}{\pi LD^2 \left(1 + \dfrac{D}{3L}\right)}$$

T(RPM) is calculated from these two equations numerically. Only data points with RPM from 5 to 200 and T% between 3 and 100 are fitted.

Table 6

| Comparative Example I | | | |
|---|---|---|---|
| Days after Manufacturing | | Viscosity at 1 RPM (cP) | Static Yield Stress (Pa) |
| 1 | | 442984 | 152 |
| 21 | | 277573 | 161 |
| 30 | | 202798 | 133 |
| 37 | | 203931 | 99 |
| 90 | | 167110 | 94 |
| 365 | | 226817 | 40 |
| Composition 11 | | | |
| Days after Manufacturing | | Viscosity at 1 RPM (cP) | Static Yield Stress (Pa) |
| 1 | | 609528 | 161 |
| 14 | | 527956 | 184 |
| 44 | | 425990 | 187 |
| 75 | | 431088 | 199 |
| 290 | | 493401 | 250 |

Example 4

Table 7 (below) describes the formulas of Compositions 12 and 13 (not claimed) and another comparative formula (Comparative Example II).

Table 7

| | Composition 12 | Composition 13 | | Comparative Example II |
|---|---|---|---|---|
| Ingredient | Wt% | | | |
| GLYCERIN | -- | 26.000000 | | 35.000000 |
| SORBITOL | 39.000000 | 13.000000 | | -- |
| SUCRALOSE | -- | -- | | 0.020000 |
| WATER | 27.824737 | 28.244737 | | 30.848737 |

(continued)

| Ingredient | Composition 12 | Composition 13 | | Comparative Example II |
|---|---|---|---|---|
| | Wt% | | | |
| SODIUM SACCHARIN | 0.400000 | 0.400000 | | 0.400000 |
| ABRASIVE SILICA | 10.000000 | 10.000000 | | 10.000000 |
| SILICA-THICKENER | 6.000000 | 6.000000 | | 7.000000 |
| AMORPHOUS SILICA | 5.000000 | 5.000000 | | 5.000000 |
| Na-LAURYL SULFATE GRANULES | 2.105263 | 2.105263 | | 2.105263 |
| Flavor | 1.500000 | 1.500000 | | 1.500000 |
| L-ARGININE | 1.500000 | 1.500000 | | 1.500000 |
| COCAMIDOPROPYL BETAINE | 1.250000 | 1.250000 | | 1.250000 |
| ZINC OXIDE | 1.000000 | 1.000000 | | 1.000000 |
| TITANIUM DIOXIDE | 0.750000 | -- | | 0.300000 |
| XANTHAN GUM | 0.300000 | 0.300000 | | 0.400000 |
| SODIUM CMC | -- | -- | | 1.100000 |
| HYDROXYETHYL CELLULOSE (HEC)* | 0.800000 | 1.000000 | | -- |
| TETRASODIUM PYROPHOSPHATE | 0.500000 | 0.500000 | | 0.500000 |
| ZINC CITRATE TRIHYDRATE | 0.500000 | 0.500000 | | 0.500000 |
| POLOXAMER 407 | 0.500000 | 0.500000 | | 0.500000 |
| BENZYL ALCOHOL | 0.400000 | 0.400000 | | 0.400000 |
| 85% PHOSPHORIC ACID | 0.350000 | 0.350000 | | 0.350000 |
| SODIUM FLUORIDE - USP, EP | 0.320000 | 0.320000 | | 0.320000 |
| Additional Colorants | -- | 0.130000 | | 0.006000 |
| *HEC having a viscosity at 2% in water, of 6000 to 7500 cps at 25 °C | | | | |

Example 5

Table 8 (below) describes the percentage change in viscosity loss and loss of static yield stress for two exemplary compositions (Compositions 12 and 13) and a comparative composition (Comparative Example II). Viscosity and Static Yield Stress were calculated in accordance with the methods described in Example 3 herein.

Table 8

| Composition | Viscosity Loss (1 Year) | Loss of Static Yield Stress (After 1 Year) |
|---|---|---|
| Composition 12 | 19 % | Stable |
| Composition 13 | 21 % | Stable |
| Comparative Example II | 49 % | 73 % |

Example 6

Table 9 (below) describes another exemplary backbone for oral care compositions comprising - *inter alia* - a basic amino acid in free or salt form (e.g. L-arginine); and a combination of zinc ion sources.

Table 9

| Ingredient | Wt.% |
|---|---|
| L-Arginine | 1.50 |
| Zinc Citrate Trihydrate | 0.50 |
| Sodium Fluoride | 0.32 |
| Non-ionic surfactant | 0.50 |
| Alkali Phosphate salt | 0.50 |
| Zinc Oxide | 1.00 |
| Saccharin | 0.40 |
| Colorant | 0.75 |
| Silica Abrasives | 15.00 |
| Silica thickener | 7.50 |
| Anionic surfactant | 2.00 |
| Flavoring agent | 1.52 |
| Preservative | 0.40 |
| Amphoteric surfactant | 1.25 |

Added to this backbone were the various combinations of water, a nonionic cellulose ether (hydroxyethylcellulose [HEC]); and a polysaccharide gum (xanthan gum), described in Table 10 (below).

Table 10

| Composition | Water | Glycerin | Xanthan Gum | HEC** |
|---|---|---|---|---|
| | Wt.% | | | |
| 14* | 32.78 | 32.78 | 0.3 | 1 |
| 15* | 33.03 | 33.03 | 0.3 | 0.5 |
| 16* | 32.83 | 32.83 | 0.2 | 1 |
| 17* | 32.86 | 32.86 | 0.35 | 0.8 |
| 18* | 32.91 | 32.91 | 0.45 | 0.6 |
| 19* | 32.71 | 32.71 | 0.25 | 1.2 |
| 20* | 33.08 | 33.08 | 0.55 | 0.15 |
| 21* | 32.93 | 32.93 | 0.5 | 0.5 |
| 22* | 32.88 | 32.88 | 0.5 | 0.6 |
| 23* | 32.91 | 32.91 | 0.55 | 0.5 |
| **HEC having a viscosity, measured at 2% in water at 25 °C, of 4500 to 6500 cps<br>* not claimed | | | | |

Toothpastes were prepared from each of the combinations described in Table 10 (above) by first creating a gel comprising water, glycerin, xanthan gum and hydroxyethylcellulose (HEC); and then combining each gel with the remaining components (see Table 9) in a Ross double planetary mixer. The rheological profiles of both the gel pre-mixes and the toothpaste end products are evaluated.

Table 11 (below) describes exemplary gel pre-mixes (gel pre-mixes are not claimed). Although not shown in Table 11, the gel pre-mixes also included water and glycerin in equal amounts.

Table 11

| Composition | Xanthan Gum | HEC** |
|:---:|:---:|:---:|
| | Wt.% | |
| A | 0.9 | 0.2 |
| B | 0.7 | 0.35 |
| C | 0.6 | 0.5 |
| D | 0.55 | 0.6 |
| E | 0.5 | 0.7 |
| F | 0.45 | 0.8 |
| G | 0.7 | 0.7 |
| H | 0.8 | 0.5 |
| I | 1 | 0.3 |
| J | 1.2 | 0.15 |
| K | 1.1 | 0 |
| L | 0.9 | 0 |
| M | 1.05 | 0.15 |
| N | 0.9 | 0.1 |
| O | 0.85 | 0.3 |
| P | 0.9 | 0.4 |
| Q | 0.7 | 0.5 |
| R | 0.45 | 0.35 |
| S | 0.3 | 0.4 |
| T | 0.25 | 1.2 |
| U | 0.4 | 0.6 |
| V | 0.55 | 0.15 |
| W | 0.68 | 0.25 |
| X | 0.6 | 0.45 |
| **HEC having a viscosity, measured at 2% in water at 25 °C, of 4500 to 6500 cps | | |

Example 7

The processability of the gel pre-mixes described in Table 11 (above) is evaluated by flowing a sample gel through a gel tank exposed to negative pressure (at room temperature), and characterizing each sample in terms of "drainage time" and "left-overs". In these experiments 3 kg of the gel is being drained from a tank of 21 cm in diameter through a 1 mm wide bottom opening at negative pressure of (-)0.95 bar. "Drainage time" is defined as the time elapsed between the start of the flow from the inlet and the time at which air begins to enter the outlet (at the bottom) of the gel tank. The material remaining in the gel tank is collected and weighed to determine "left-overs". The results of these evaluations are described in Table 12 (below).

Table 12

| Composition | Left-overs (g) | Drainage Time (min) |
|:---:|:---:|:---:|
| A | 473 | 24 |
| B | 494 | 13 |

**13**

(continued)

| Composition | Left-overs (g) | Drainage Time (min) |
|---|---|---|
| c | 335 | 38.5 |
| D | 341 | 45 |
| E | 846 | 65 |
| F | 765 | 85 |
| G | 1062.5 | 78 |
| H | 533 | 60 |
| I | 607 | 40 |
| J | 879 | 48 |
| K | 642 | 13 |
| L | 384 | 8.5 |
| M | 637 | 28 |
| N | 409.5 | 15.5 |
| O | 474 | 33 |
| P | 591 | 46 |
| Q | 370 | 33 |
| R | 128 | 13 |
| S | 92.6 | 4.5 |
| T | 1106 | n/a |
| U | 347 | 28 |
| V | 101 | 2 |

Example 8

Static Yield Stress (YS) of toothpastes prepared by adding the combinations described in Table 10 (above) to the backbone described in Table 9 (above) was calculated as follows. Measurements are performed on ARG2 rheometer by TA Instrument using a cylindrical cup and vane upper geometry. The measurements were performed in the same containers, in which the samples were aged. Those were standard 50 cc centrifuge tubes, available from VWR, into which the samples were placed at the time of preparation. Then the samples were consolidated by centrifuging at low rotations in the centrifuge so as to remove air pockets and aged directly in these tubes at room temperature (RT) or at 49 °C for up to 2 months. Before the measurements the heated tubes were allowed to cool for 2 hours at RT and then inserted directly into the cup of the rheometer. To avoid the tubes mobility in the cup, they were wrapped with a thin layer of tape. The vane was inserted into the tubes and samples measured directly inside them. The measurement procedure closely mimicked the one described above for Brookfield viscometer, i.e., a constant shear rate of 0.05 sec$^{-1}$ was applied for 400 sec and followed by shear rate sweeps up and down from 0.1 to 30 sec$^{-1}$. Here shear rate is calculated from angular velocity of the vane, $\Omega$, following TA Instruments conventions as follows:

$$S = \frac{1+k^2}{1-k^2}\Omega$$

where k is the ratio of the diameter of the vane to the diameter of the tube. Torque, T, on the shaft of the vane was measured. Yield stress was calculated by fitting T($\Omega$) with the theoretical function calculated assuming Casson constitutive equation and implicitly given by the following equation:

$$\Omega = \frac{1}{2}\int_{SW0}^{SW}\frac{\left(S^n - YS^n\right)^{l/n}}{HSV * S}dS$$

where HSV (high-shear viscosity limit) is another fitting parameter, n=0.2, and SW is the stress on the imaginary wall encompassing the vane which is estimated as follows:

$$SW = \frac{2T}{\pi LD^2(1+\dfrac{D}{3L})}$$

and SW0 is the largest of the two values: YS and $k^2SW$. Note that the exponent n used to process these data is different from the one used to process Brookfield data above (0.2 instead of 0.3) to accommodate a wide range of shear rates as measured by a rheometer.

The results are described below in Table 13.

Table 13

| Composition | YS (Pa) |
|---|---|
| 14 | 40.2 |
| 15 | 9.5 |
| 16 | 17.6 |
| 17 | 30.3 |
| 18 | 39.1 |
| 19 | 35.8 |
| 20 | 24.9 |
| 21 | 27.7 |
| 22 | 33.2 |
| 23 | 39.1 |

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

**Claims**

1. An oral care composition comprising:

    a. a basic amino acid in free or salt form wherein the basic amino acid is selected from arginine, lysine, and a combination thereof;
    b. a combination of zinc ion sources; and
    c. a thickening system comprising:

        i. from 0.1 wt.% to 0.4 wt.% of a nonionic cellulose ether which is hydroxyethylcellulose; and
        ii. from 0.25 wt.% to 0.9 wt.% of a polysaccharide gum which is xanthan gum.

2. The oral care composition according to claim 1, wherein the nonionic cellulose ether has a viscosity, measured at 2% in water at 25 °C, of from 4500 to 7500 cps, or from 4500 to 6500 cps, or from 6000 to 7500 cps. wherein the viscosity is measured on a Brookfield HADV2 viscometer using a V74 vane spindle, wherein the RPM of the spindle is swept from 200 to 0.5 in 12 steps with 10 seconds per step and wherein the viscosity reading reported is taken at RPM=1.

3. The oral care composition according to any foregoing claim, comprising

(i) from 0.1 wt.% to 0.3 wt.% of hydroxyethylcellulose and/or from 0.3 wt.% to 0.8 wt.% of xanthan gum; or
(ii) 0.1 wt.%, 0.15 wt.%, 0.2 wt.%, 0.25 wt.% or 0.3 wt.% of hydroxyethylcellulose; or
(iii) from 0.3 wt.% to 0.9 wt.% of xanthan gum; or
(iv) from 0.4 wt.% to 0.9 wt.% of xanthan gum; or
(v) from 0.5 wt.% to 0.9 wt.% of xanthan gum; or
(vi) from 0.6 wt.% to 0.9 wt.% of xanthan gum; or
(vii) 0.3 wt.%, 0.4 wt.%, 0.5 wt.%, 0.6 wt.%, 0.7 wt.%, 0.8 wt.%, 0.9 wt.% of xanthan gum; or
(viii) from 0.6 wt.% to 0.8 wt.% of xanthan gum.

4. The oral care composition according to any foregoing claim, wherein the thickening system further comprises from 5 wt.% to 10 wt.% silica.

5. The oral care composition according to any foregoing claim, wherein the thickening system comprises from 0.5 wt.% to 15 wt.% of the oral care composition.

6. The oral care composition according to any foregoing claim, wherein hydroxyethylcellulose and xanthan gum are present in a weight ratio of from 1:1 to 1:10, or from 1:2 to 1:10, or 1:3 to 1:10, or in a weight ratio selected from 1:3, 1:4, 1:5, 1:6, 1:7, 1:8, and 1:9.

7. The oral care composition according to any foregoing claim, wherein the combination of zinc ion sources comprises zinc oxide and zinc citrate, preferably wherein the weight ratio of zinc oxide to zinc citrate is 2:1, further preferred wherein the zinc citrate is in an amount of 0.5 wt.% and zinc oxide is present in an amount of 1.0 wt.% based on the total weight of the oral care composition.

8. The oral care composition according to any foregoing claim, wherein the amino acid is arginine, and is present at 1.5 wt.%, 5 wt.%, or 8 wt.%, of the oral care composition.

9. The oral care composition according to any foregoing claim, further comprising a fluoride ion source selected from sodium fluoride, sodium monofluorophosphate, and stannous fluoride, preferably wherein the fluoride ion source comprises stannous fluoride.

10. The oral care composition according to any foregoing claim, comprising:

a. 1.0 wt.% zinc oxide
b. 0.5 wt.% zinc citrate
c. 1.5 wt.% L-arginine
d. from 0.3 wt.% to 0.8 wt.% of xanthan gum; and
e. from 0.1 wt.% to 0.5 wt.% of hydroxyethylcellulose.

11. The oral care composition according to any foregoing claim, wherein the oral care composition is in a form selected from: a toothpaste, a mouthwash, and a gel.

12. Oral care composition according to any foregoing claim for use in a method to:

i. reduce or inhibit formation of dental caries,
ii. reduce or inhibit demineralization of the teeth,
iii. reduce hypersensitivity of the teeth,
iv. reduce or inhibit gingivitis,
v. promote healing of sores or cuts in the mouth,
vi. reduce levels of acid producing bacteria,
vii. to increase relative levels of arginolytic bacteria,
viii. inhibit microbial bio film formation in the oral cavity,
ix. reduce plaque accumulation,

x. reduce erosion of the teeth, and/or

xi. clean the teeth and oral cavity

wherein said method comprises applying an effective amount of said oral care composition to the oral cavity of a subject in need thereof.

**Patentansprüche**

1. Mundpflegezusammensetzung, umfassend:

a. eine basische Aminosäure in freier oder Salzform, wobei die basische Aminosäure ausgewählt ist aus Arginin, Lysin und einer Kombination davon;
b. eine Kombination von Zinkionenquellen; und
c. ein Verdickungssystem, umfassend:

i. von 0,1 Gew.-% bis 0,4 Gew.-% eines nichtionischen Celluloseethers, der Hydroxyethylcellulose ist; und
ii. von 0,25 Gew.-% bis 0,9 Gew.-% eines Polysaccharidgummis, der Xanthan ist.

2. Mundpflegezusammensetzung gemäß Anspruch 1, wobei der nichtionische Celluloseether eine Viskosität von 4500 bis 7500 cps, oder von 4500 bis 6500 cps, oder von 6000 bis 7500 cps, gemessen bei 2% in Wasser bei 25 °C, aufweist, wobei die Viskosität mit einem Brookfield-HADV2-Viskosimeter unter Verwendung einer V74-Flügelspindel gemessen wird, wobei die U/min der Spindel in 12 Schritten mit 10 Sekunden pro Schritt von 200 auf 0,5 geändert wird und wobei die Viskositätsanzeige bei U/min = 1 abgelesen wird.

3. Mundpflegezusammensetzung gemäß einem beliebigen vorhergehenden Anspruch, umfassend

(i) von 0,1 Gew.-% bis 0,3 Gew.-% Hydroxyethylcellulose und/oder von 0,3 Gew.-% bis 0,8 Gew.-% Xanthan; oder
(ii) 0,1 Gew.-%, 0,15 Gew.-%, 0,2 Gew.-%, 0,25 Gew.-% oder 0,3 Gew.-% Hydroxyethylcellulose; oder
(iii) von 0,3 Gew.-% bis 0,9 Gew.-% Xanthan; oder
(iv) von 0,4 Gew.-% bis 0,9 Gew.-% Xanthan; oder
(v) von 0,5 Gew.-% bis 0,9 Gew.-% Xanthan; oder
(vi) von 0,6 Gew.-% bis 0,9 Gew.-% Xanthan; oder
(vii) 0,3 Gew.-%, 0,4 Gew.-%, 0,5 Gew.-%, 0,6 Gew.-%, 0,7 Gew.-%, 0,8 Gew.-%, 0,9 Gew.-% Xanthan; oder
(viii) von 0,6 Gew.-% bis 0,8 Gew.-% Xanthan.

4. Mundpflegezusammensetzung gemäß einem beliebigen vorhergehenden Anspruch, wobei das Verdickungssystem weiterhin von 5 Gew.-% bis 10 Gew.-% Siliciumdioxid umfasst.

5. Mundpflegezusammensetzung gemäß einem beliebigen vorhergehenden Anspruch, wobei das Verdickungssystem von 0,5 Gew.-% bis 15 Gew.-% der Mundpflegezusammensetzung umfasst.

6. Mundpflegezusammensetzung gemäß einem beliebigen vorhergehenden Anspruch, wobei Hydroxyethylcellulose und Xanthan in einem Gewichtsverhältnis von 1:1 bis 1:10, oder von 1:2 bis 1:10, oder 1:3 bis 1:10, oder in einem Gewichtsverhältnis ausgewählt aus 1:3, 1:4, 1:5, 1:6, 1:7, 1:8 und 1:9 vorliegen.

7. Mundpflegezusammensetzung gemäß einem beliebigen vorhergehenden Anspruch, wobei die Kombination von Zinkionenquellen Zinkoxid und Zinkcitrat umfasst, vorzugsweise wobei das Gewichtsverhältnis von Zinkoxid zu Zinkcitrat 2:1 beträgt, weiterhin vorzugsweise wobei das Zinkcitrat in einer Menge von 0,5 Gew.-% und Zinkoxid in einer Menge von 1,0 Gew.-%, bezogen auf das Gesamtgewicht der Mundpflegezusammensetzung, vorliegen.

8. Mundpflegezusammensetzung gemäß einem beliebigen vorhergehenden Anspruch, wobei die Aminosäure Arginin ist und in einer Menge von 1,5 Gew.-%, 5 Gew.-% oder 8 Gew.-% der Mundpflegezusammensetzung vorliegt.

9. Mundpflegezusammensetzung gemäß einem beliebigen vorhergehenden Anspruch, weiterhin umfassend eine Fluoridionenquelle, ausgewählt aus Natriumfluorid, Natriummonofluorphosphat und Zinnfluorid, vorzugsweise wobei die Fluoridionenquelle Zinnfluorid umfasst.

10. Mundpflegezusammensetzung gemäß einem beliebigen vorhergehenden Anspruch, umfassend:

    a. 1,0 Gew.-% Zinkoxid
    b. 0,5 Gew.-% Zinkcitrat
    c. 1,5 Gew.-% L-Arginin
    d. von 0,3 Gew.-% bis 0,8 Gew.-% Xanthan; und
    e. von 0,1 Gew.-% bis 0,5 Gew.-% Hydroxyethylcellulose.

11. Mundpflegezusammensetzung gemäß einem beliebigen vorhergehenden Anspruch, wobei die Mundpflegezusammensetzung in einer Form vorliegt, die ausgewählt ist aus: einer Zahnpasta, einer Mundspülung und einem Gel.

12. Mundpflegezusammensetzung gemäß einem beliebigen vorhergehenden Anspruch zur Verwendung in einem Verfahren um:

    i. Bildung von Zahnkaries zu reduzieren oder zu verhindern,
    ii. Demineralisierung der Zähne zu reduzieren oder zu verhindern,
    iii. Überempfindlichkeit der Zähne zu reduzieren,
    iv. Gingivitis zu reduzieren oder zu verhindern,
    v. Heilung von Wunden oder Schnitten im Mund zu fördern,
    vi. den Gehalt an säureproduzierenden Bakterien reduzieren,
    vii. relative Gehalte an arginolytischen Bakterien zu erhöhen,
    viii. mikrobielle Biofilmbildung in der Mundhöhle zu verhindern,
    ix. Plaque-Akkumulation zu reduzieren,
    x. Erosion der Zähne zu reduzieren, und/oder
    xi. Zähne und die Mundhöhle zu reinigen,

wobei das Verfahren Auftragen einer wirksamen Menge der Mundpflegezusammensetzung in die Mundhöhle eines Subjekts, das diese benötigt, umfasst.

**Revendications**

1. Composition de soins bucco-dentaires comprenant :

    a. un acide aminé basique sous forme libre ou saline dans lequel l'acide aminé basique est choisi parmi l'arginine, la lysine et une combinaison de celles-ci ;
    b. une combinaison de sources d'ions zinc ; et
    c. un système épaississant comprenant :

        i. de 0,1 % en poids à 0,4 % en poids d'un éther de cellulose non ionique qui est l'hydroxyéthylcellulose ; et
        ii. de 0,25 % en poids à 0,9 % en poids d'une gomme polysaccharidique qui est la gomme xanthane.

2. Composition de soins bucco-dentaires selon la revendication 1, dans laquelle l'éther de cellulose non ionique a une viscosité, mesurée à 2 % dans de l'eau à 25 °C, de 4 500 à 7 500 cps, ou de 4 500 à 6 500 cps, ou de 6 000 à 7 500 cps, la viscosité étant mesurée sur un viscosimètre Brookfield HADV2 en utilisant un mobile à ailettes, dans lequel la RPM est balayée de 200 à 0,5 en 12 étapes avec 10 secondes par étape et la lecture de viscosité rapportée étant effectuée à RPM = 1.

3. Composition de soins bucco-dentaires selon l'une quelconque des revendications précédentes, comprenant

    (i) de 0,1 % en poids à 0,3 % en poids d'hydroxyéthylcellulose et/ou de 0,3 % en poids à 0,8 % en poids de gomme xanthane ; ou
    (ii) 0,1 % en poids, 0,15 % en poids, 0,2 % en poids, 0,25 % en poids ou 0,3 % en poids d'hydroxyéthylcellulose ; ou
    (iii) de 0,3 % en poids à 0,9 % en poids de gomme xanthane ; ou
    (iv) de 0,4 % en poids à 0,9 % en poids de gomme xanthane ; ou
    (v) de 0,5 % en poids à 0,9 % en poids de gomme xanthane ; ou
    (vi) de 0,6 % en poids à 0,9 % en poids de gomme xanthane ; ou
    (vii) 0,3 % en poids, 0,4 % en poids, 0,5 % en poids, 0,6 % en poids, 0,7 % en poids, 0,8 % en poids, 0,9 % en poids de gomme xanthane ; ou

(viii) de 0,6 % en poids à 0,8 % en poids de gomme xanthane.

4. Composition de soins bucco-dentaires selon l'une quelconque des revendications précédentes, dans laquelle le système épaississant comprend en outre de 5 % en poids à 10 % en poids de silice.

5. Composition de soins bucco-dentaires selon l'une quelconque des revendications précédentes, dans laquelle le système épaississant comprend de 0,5 % en poids à 15 % en poids de la composition de soins bucco-dentaires.

6. Composition de soins bucco-dentaires selon l'une quelconque des revendications précédentes, dans laquelle l'hydroxyéthylcellulose et la gomme xanthane sont présentes selon un rapport pondéral de 1:1 à 1:10, ou de 1:2 à 1:10, ou de 1:3 à 1:10, ou selon un rapport pondéral choisi parmi 1:3, 1:4, 1:5, 1:6, 1:7, 1:8 et 1:9.

7. Composition de soins bucco-dentaires selon l'une quelconque des revendications précédentes, dans laquelle la combinaison de sources d'ions zinc comprend de l'oxyde de zinc et du citrate de zinc, de préférence dans laquelle le rapport pondéral de l'oxyde de zinc au citrate de zinc est de 2:1, de préférence encore dans laquelle le citrate de zinc est en une quantité de 0,5 % en poids et l'oxyde de zinc est présent en une quantité de 1,0 % en poids par rapport au poids total de la composition de soins bucco-dentaires.

8. Composition de soins bucco-dentaires selon l'une quelconque des revendications précédentes, dans laquelle l'acide aminé est l'arginine et est présent à raison de 1,5 % en poids, 5 % en poids ou 8 % en poids de la composition de soins bucco-dentaires.

9. Composition de soins bucco-dentaires selon l'une quelconque des revendications précédentes, comprenant en outre une source d'ions fluorure choisie parmi le fluorure de sodium, le monofluorophosphate de sodium et le fluorure stanneux, de préférence dans laquelle la source d'ions fluorure comprend du fluorure stanneux.

10. Composition de soins bucco-dentaires selon l'une quelconque des revendications précédentes, comprenant :

    a. 1,0 % en poids d'oxyde de zinc
    b. 0,5 % en poids de citrate de zinc
    c. 1,5 % en poids de L-arginine
    d. de 0,3 % en poids à 0,8 % en poids de gomme xanthane ; et
    e. de 0,1 % en poids à 0,5 % en poids d'hydroxyéthylcellulose.

11. Composition de soins bucco-dentaires selon l'une quelconque des revendications précédentes, dans laquelle la composition de soins bucco-dentaires est sous une forme choisie parmi : un dentifrice, un bain de bouche et un gel.

12. Composition de soins bucco-dentaires selon l'une quelconque des revendications précédentes pour une utilisation dans un procédé pour :

    i. réduire ou inhiber la formation de caries dentaires,
    ii. réduire ou inhiber la déminéralisation des dents,
    iii. réduire l'hypersensibilité des dents,
    iv. réduire ou inhiber la gingivite,
    v. favoriser la guérison de plaies ou de coupures dans la bouche,
    vi. réduire les niveaux des bactéries productrices d'acide,
    vii. pour augmenter les niveaux relatifs des bactéries arginolytiques,
    viii. inhiber la formation de biofilm microbien dans la cavité buccale,
    ix. réduire l'accumulation de plaque,
    x. réduire l'érosion des dents, et/ou
    xi. nettoyer les dents et la cavité buccale

dans lequel ledit procédé comprend l'application d'une quantité efficace de ladite composition de soins bucco-dentaires à la cavité buccale d'un sujet en ayant besoin.

**EP 3 344 219 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2015320654 A1
- US 3535421 A, Briner
- US 4885155 A, Parran, Jr.
- US 3678154 A, Widder
- US 4842847 A
- US 4866161 A, Sikes
- WO 2013089734 A1